# EUROPEAN PATENT APPLICATION

(11) **EP 4 407 627 A1**
(43) Date of publication of application: **31.07.2024**
(21) Application number: 23154061.8
(22) Date of filing: 30.01.2023
(51) Int. Cl.: G16H 10/20, G16H 50/20, A61B 5/00, A61B 5/16

(54) **NUMEROSITY ESTIMATION IMPAIRMENT MEASUREMENT SYSTEM**

(71) Applicant: ECOLE POLYTECHNIQUE FEDERALE DE LAUSANNE (EPFL), 1015 Lausanne (CH)
(72) Inventor: BLANKE, Olaf, 1260 NYON (CH); ALBERT, Louis, 01210 FERNEY VOLTAIRE (FR); HERBELIN, Bruno, 01210 FERNEY VOLTAIRE (FR); BERNASCONI, Fosco, 1260 NYON (CH); POTHEEGADOO, Jevita, 1113 ST-SAPHORIN-SUR-MORGES (CH)
(74) Representative: Byrne, Declan

(57) **Abstract**

The present invention concerns a numerosity estimation impairment measurement system for diagnostic/prognosis of a neurological disorder. The system comprises an electronic display device configured to present 3D scene representations/images comprising a plurality of 3D virtual visual stimulus to be evaluated by a user; a 3D scene generating device configured to provide 3D scene representations/images comprising the plurality of 3D virtual visual stimulus to the electronic display device; an electronic input device configured to receive a user input representing a user indicated numerosity of the presented plurality of virtual visual stimulus of the 3D scene representation/image; electronic storage means configured to store a predetermined numerosity of the plurality of 3D virtual visual stimulus and the user input representing the user indicated numerosity; and electronic calculation means configured to determine a numerosity estimation impairment by determining an overestimation in numerosity estimations by the user using the predetermined numerosity and the associated user input representing the user indicated numerosity.

## Description

### FIELD OF THE INVENTION

The present invention relates to a numerosity estimation impairment measurement system.

The presented method and system provide a solution for measuring and quantifying an impairment in numerosity estimation in human subjects. This method and system finds application, but is not limited to, as an indicator of a specific cognitive impairment in patients with neurological (or psychiatric) disorders enabling the diagnostic and/or the prognosis of the evolution of their illness.

### BACKGROUND

The rapid estimation of the number of elements in a visual stimulus, or numerosity estimation (NE), is an innate cognitive ability of humans. For more than four visual elements, an exact count is not perceived rapidly (i.e. without counting). Moreover, numerosity estimation is known to be imprecise and affected by several factors. Recent technological and scientific findings of the Inventors show an association between the over-estimation of the number of human elements perceived (as opposed to non-human elements) and the sensitivity to experience Presence Hallucination (PH).

This is observed in both healthy participants when exposed to conditions inducing PH (e.g., linked to robotically induced PH, see for example US patent applications US2016/0176053 and US2018/0177462, Blanke et al), and in patients who experience spontaneous PH of neurological origin (for example, in patients with Parkinson's disease).

Classical evidence from behavioral research on numerosity estimation (NE) for different visual stimuli (see, for example, Burr, D. C., Anobile, G., & Arrighi, R. (2017), Psychophysical evidence for the number sense, Philosophical Transactions of the Royal Society of London, Series B, Biological Sciences, 373(1740)) has shown that healthy participants have the ability to rapidly estimate the number of elements in a visual stimulus. This was demonstrated experimentally by flashing visual stimuli on a screen (2D dots, short duration) and asking participants to report the number of dots they perceived. A classical effect, confirmed in many studies, is that participants overestimate the number of visual stimuli presented (i.e. the number of dots shown) in the lower part of the estimation range (from 5 to 20 dots, upper limit varies across studies). An underestimation is then observed for higher numerosities. Below this number (from 1 to 4 elements), visual numerosity involves another process called subitizing which is a rapid, confident, and accurate report of the number of elements presented for short durations.

The Inventors have previously shown that presence hallucinations (PH) are associated with systematic overestimation errors in numerosity estimation (NE) of people felt-close when judging small groups of 2 to 4 people (Blanke, O., Pozeg, P., Hara, M., Heydrich, L., Serino, A., Yamamoto, A., Higuchi, T., Salomon, R., Seeck, M., Landis, T., Arzy, S., Herbelin, B., Bleuler, H., & Rognini, G. (2014), Neurological and robot-controlled induction of an apparition, Current Biology: CB, 24(22), 2681-2686). Here the numerosity estimation (NE) of people felt-close was based on a robotic procedure. Prior to performing the robotic procedure inducing presence hallucinations (riPH), participants were asked to look at several people in the room, where the presence hallucinations (PH) experiment was carried out, and were told that during the experiment these people could have left the room or not. Individuals were then blindfolded and asked to perform the robotic task and, during the robotic stimulation, asked to estimate the number of people felt-close in the room. These early results suggested that conditions inducing presence hallucinations (PH) result in a higher numerosity estimation (NE) of people felt-close.

Testing the numerosity estimation (NE) of people felt-close in such conditions, however, is practically challenging because it requires the availability of two to four trained people/experimenters at all times (entering the room), which is not feasible nor practical in clinics. This also induces methodological flaws, as this visual stimulation is hardly controlled (i.e., position, movement, and other social-affective aspects of the people's "behavior").

Importantly, such numerosity estimation (NE) has methodological flaws and is insufficiently controlled, as the number of shown people (entering the room), their position, their movement, the social-affective aspects of their "behavior", the number of repeated trials, and the addition of control conditions is very limited, strongly reducing the applicability and robustness of numerosity estimation (NE) of people felt-close as an implicit measure of presence hallucinations (PH).

A goal of the present invention is to provide a solution to these inconveniences, and in particular, to provide a method and system that improves the applicability and robustness of numerosity estimation (NE) of people as an implicit measure of presence hallucinations (PH), and as an indicator of a specific cognitive impairment in patients with neurological (or psychiatric) disorders enabling the diagnostic and/or the prognosis of the evolution of their illness.

### SUMMARY

It is therefore one aspect of the present disclosure to provide a numerosity estimation impairment (NEI) measurement system according to claim 1 that addresses the above-mentioned inconveniences and needs.

Specific embodiments and other advantageous features can be found in the dependent claims.

Specifically, following the procedure and using the system of the present disclosure allows estimating if a human subject is systematically over-estimating the number of people shown in a visual stimulus, as compared to the estimation by the human subject of a number of objects shown in a visual stimulus. This specific impairment in numerosity estimation can then be quantified into a numerical score that can, for example, be combined with other information on a patient to establish a diagnosis or establish a prognosis of the evolution of a neurodegenerative disease, that is, over-estimation is linked to presence hallucination (PH), which is linked to faster cognitive decline and to dementia.

The method and system of the present disclosure include (i) specific techniques to generate visual stimuli for the numerosity estimation of visual humans in a visual room, (ii) procedures to enable the comparison of numerosity estimation of visual humans with numerosity estimation of visual objects, (iii) procedures for presenting the stimuli in the appropriate testing conditions and reliability (e.g. on computer, television, virtual reality system, tablet, phone, etc.), and (iv) methods for computing a score that can be used as an indicator of the subjects' impairment in numerosity estimation of human figures vs. objects (i.e. systematic over-estimation).

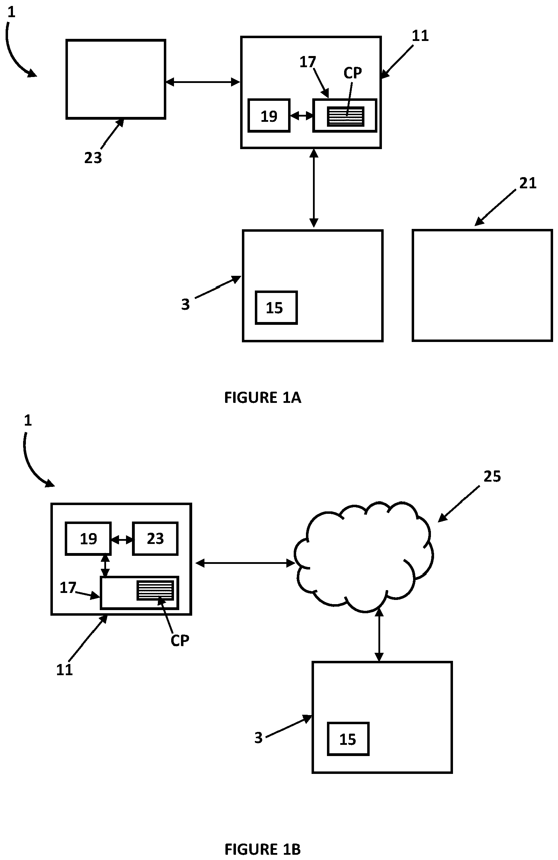

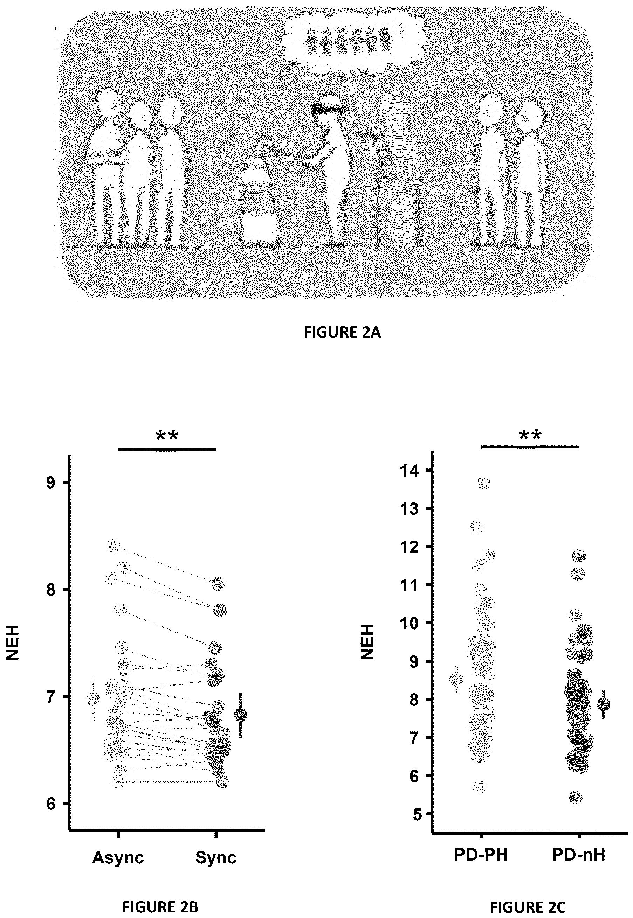

Additionally, the Inventors merged or combined their robotic platform with the system of the present disclosure implemented, for example, through virtual reality (VR) technology (see Figure 1) and designed a VR-enhanced robotics platform, which was able to overcome the previously mentioned limitations of robotic platform. Results show that the robotic condition inducing presence hallucinations (PH) (using the robotic platform) is associated with higher numerosity estimation (NE) of virtual people in virtual reality (VR) (but not for virtual objects), as if the hallucinated felt presence (induced by the robotic procedure) was added to the people the participants saw in the virtual environment (see results for the robotic platform shown in Figure 2C). These results indicate that numerosity estimation of virtual people in virtual reality (VR) can be used as an implicit and quantitative measure of presence hallucinations (PH) for many numerosities, for repeated trials, and across several experimental conditions.

Furthermore, the Inventors investigated if patients with Parkinson's disease (PD) suffering from presence hallucinations (PH) (independent of the robotic procedure) are more prone to numerosity estimation (NE) overestimation of humans (due to their susceptibility to presence hallucinations (PH)) using the system of the present disclosure. The Inventors conducted an online experiment. In this online experiment, the participants were asked to perform the same numerosity estimation (NE) task (as carried out for the VR-robotic platform), but online, on a 2D display and without any robotic stimulation by the robotic platform. Based on obtained results using the VR-robotics platform, the Inventors predicted that patients with Parkinson's disease (PD) and presence hallucinations (PH) are characterized by stronger numerosity estimation (NE) overestimation for humans, compared to patients with Parkinson's disease (PD) but without presence hallucinations (PH), and that this effect would be weaker or absent when objects instead of humans are shown to the patient or participant.

Results from this online study using the system of the present disclosure corroborate the Inventor's hypothesis that Parkinson's disease (PD) patients with disease-related presence hallucinations (PH) have a stronger numerosity estimation (NE) overestimation bias for humans, suggesting that the combination of minor hallucinations (MH) and numerosity estimation (NE) can be used as reliable biomarkers for mental symptoms in Parkinson's disease (PD).

The virtual reality (VR) technology independent of the robotic procedure is similarly expected to provide the same results as the system used for the online study.

The method and system of the present disclosure allows the estimation of a person's impairment in numerosity estimation (NE), in particular the overestimation in numerosity estimation (NE) for human elements as compared to non-human elements. This score of impairment can then be included in the diagnostic and/or the prognosis of a neurodegenerative diseases for this person, and allows evaluating the risks that this person develops, for example, dementia in the future.

The approach of the Inventors extends the usual set of neuropsychological tests done in clinics to evaluate a person's cognitive impairments. Most neuropsychological tests are with pencil and paper but numerosity estimation (NE) cannot be assessed this way because it needs a rapid presentation of stimulus, for example, that is flashed to the patient, for example, flashed at a time duration <250ms. With the digitalized or computerized system of the present disclosure, these tests can now involve advanced techniques (such as 3D controlled stimuli, repeated trials with randomization, etc.).

Moreover, the method and system of the present disclosure extends standard methodology in psychophysics of numerosity estimation (NE) to the comparison of the numerosity estimation (NE) of different objects. In particular, it allows comparing numerosity estimation (NE) of human versus non-human elements. Said otherwise, the Inventors demonstrate that a specificity of the numerosity estimation (NE) can be selectively different depending on the nature of the stimuli (here human stimuli (people) compared to non-human stimuli (objects)).

The method and system resolve the problem of integrating methodologies of psychophysics of numerosity estimation (NE) for complex visual stimuli for multiple testing contexts (for example, in virtual reality (VR), or online, on a computer, tablet, etc.).

A core originality of the invention of the present disclosure is to link numerosity estimation (NE) with engineering (virtual reality (VR), robotics) and with the neuroscience of hallucinations, applied to patients with neurological disorders and/or to healthy subjects, and thus from the synergy that comes from joining these domains, presence hallucinations (PH) are linked to biases in numerosity estimation (NE) (specific for people) and together contribute to the detection and later monitoring of the evolution of a neurological disorder (for example, Parkinson's disease (PD), but not limited to Parkinson's disease (PD), and the method and system of the present disclosure could be applied to other neurological disorders or diseases).

Psychophysical experimental research (e.g., on numerosity estimation (NE)) usually requires laboratory settings, with highly constrained and controlled experimental conditions. The technique of the present disclosure is unusual in the sense that it allows testing numerosity estimation (NE) with partially controlled presentation conditions, even allowing participants to test at home on a tablet/computer screen. This goes beyond the usually expected possibilities of psychophysics tests.

The above and other objects, features and advantages of the present invention and the manner of realizing them will become more apparent, and the invention itself will best be understood from a study of the following description with reference to the attached drawings showing some preferred embodiments of the invention.

### BRIEF DESCRIPTION OF THE DRAWINGS

The accompanying drawings, which are incorporated herein and constitute part of this specification, illustrate the presently preferred embodiments of the invention, and together with the general description given above and the detailed description given below, serve to explain features of the invention.
Figures 1A and 1B schematically show exemplary numerosity estimation impairment measurement systems according to the present disclosure.
Figure 2A to 2B show VR-enhanced robotics in which the system and method of the present is combined with a robotic platform. Figure 2A schematically shows a Numerosity Estimation of Humans (NEH) task, the results of Figure 2B shows that the VR-enhanced robotics platform induces an overestimation of the Numerosity Estimation of Humans (NEH) when inducing presence hallucinations (PH) (asynchronous condition), suggesting that Numerosity Estimation of Humans (NEH) can be used as an implicit measure for presence hallucinations (PH), this is confirmed by the Inventors online study, as seen in the results of Figure 2C, in which patients with Parkinson's disease (PD) and presence hallucination (PH) have an overestimation of Numerosity Estimation of Humans (NEH) compared to patients without hallucinations.

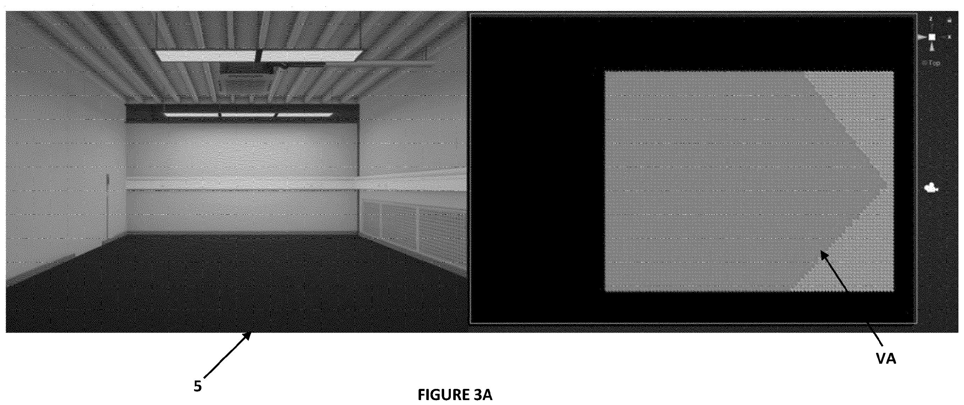

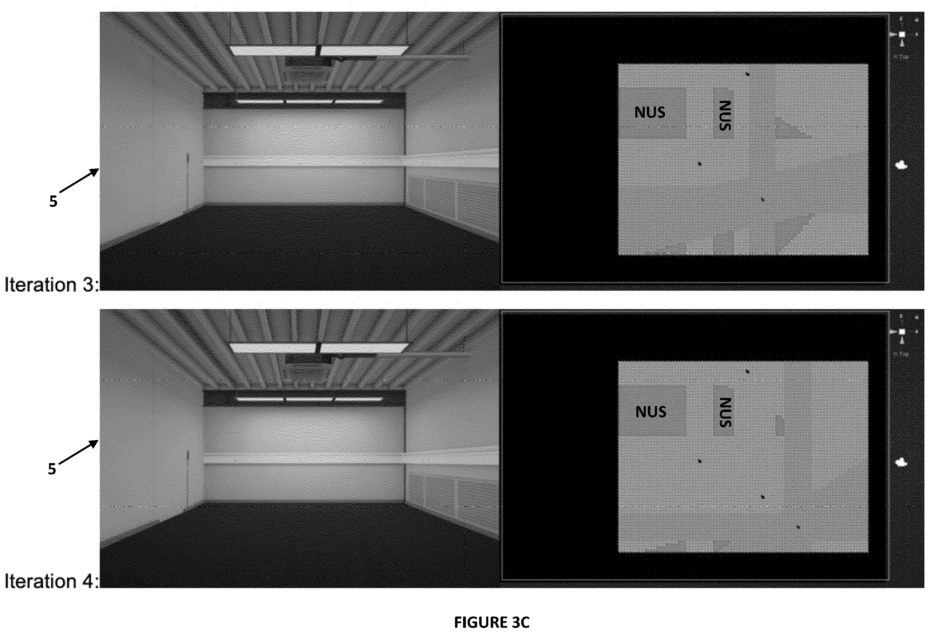

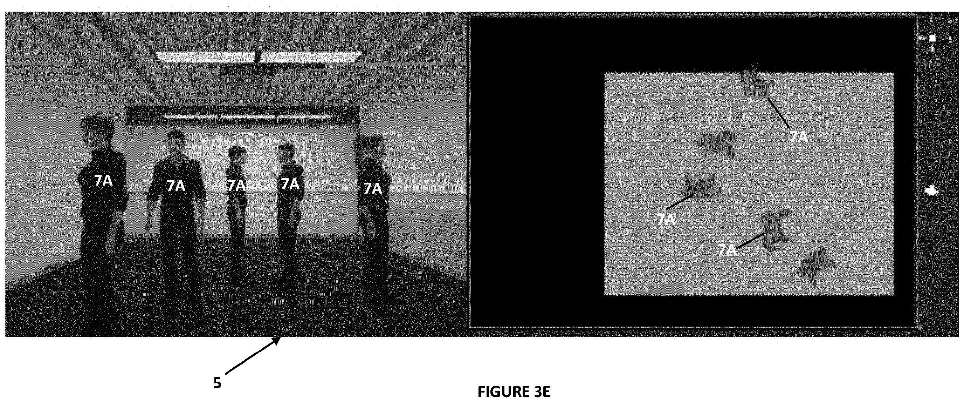

Figures 3A to 3E show a virtual room (left) generated by an algorithm of the system of the present disclosure for the repartition of elements in a virtual room for the generation of stimuli for numerosity estimation (NE), and shows steps generating element placements (right) in the virtual room, with Figure 3E showing the virtual room populated with 3D models of virtual people with different appearance but globally similar shapes.

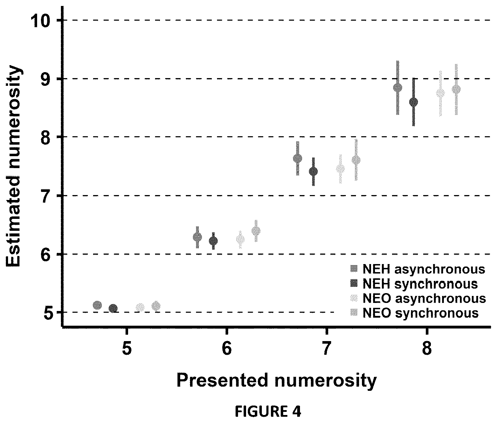

Figure 4 shows results obtained by the method and system of the present disclosure illustrating an index of bias for overestimating more people than objects which can be used by a medical doctor in relation with other neuropsychological evaluation by linking it to a proneness to presence and minor hallucinations and to a more rapidly evolving form of a cognitive decline.

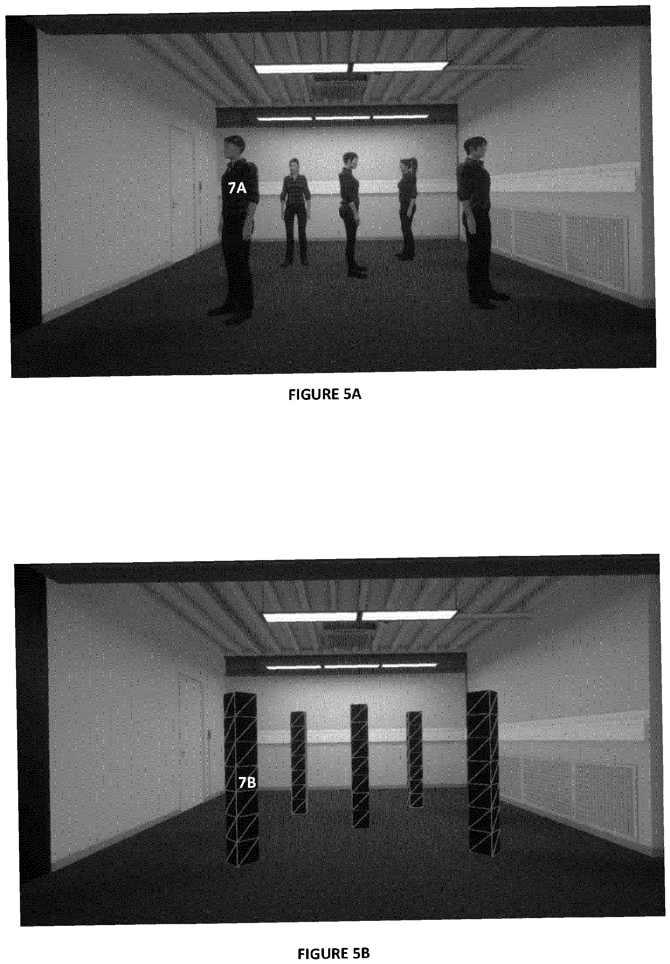

Figure 5A to 5H shows examples of possible configurations of the virtual room populated with 3D models of humans or non-human elements with different appearance but globally similar shapes.

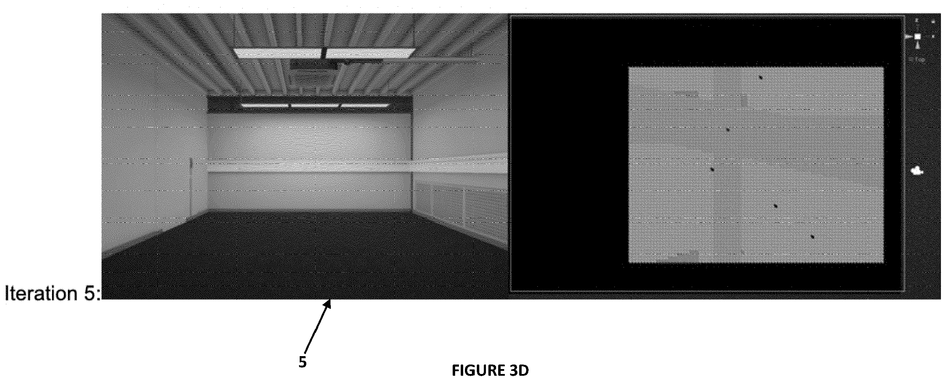

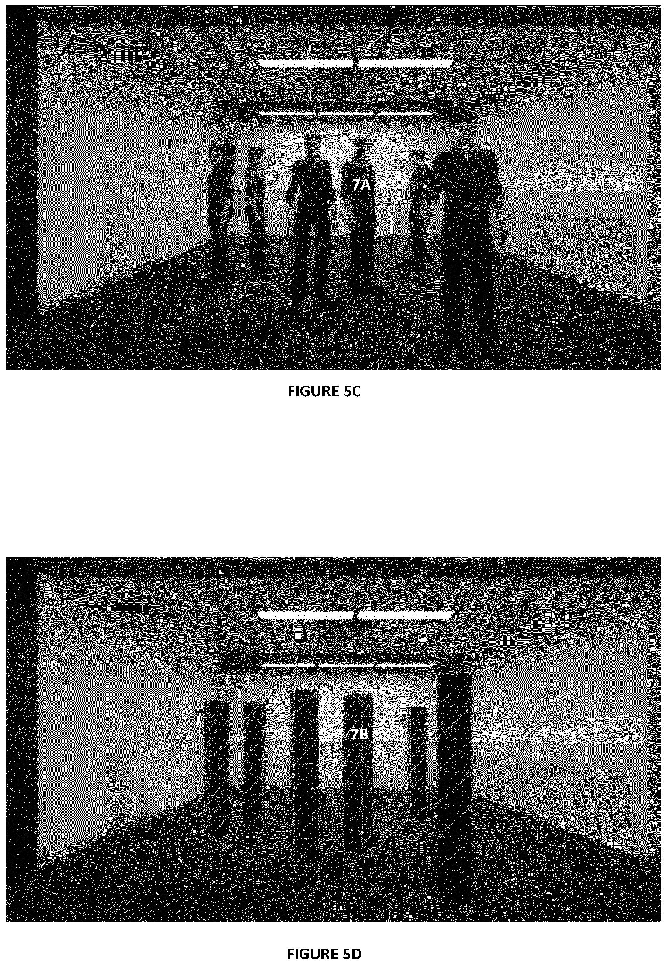

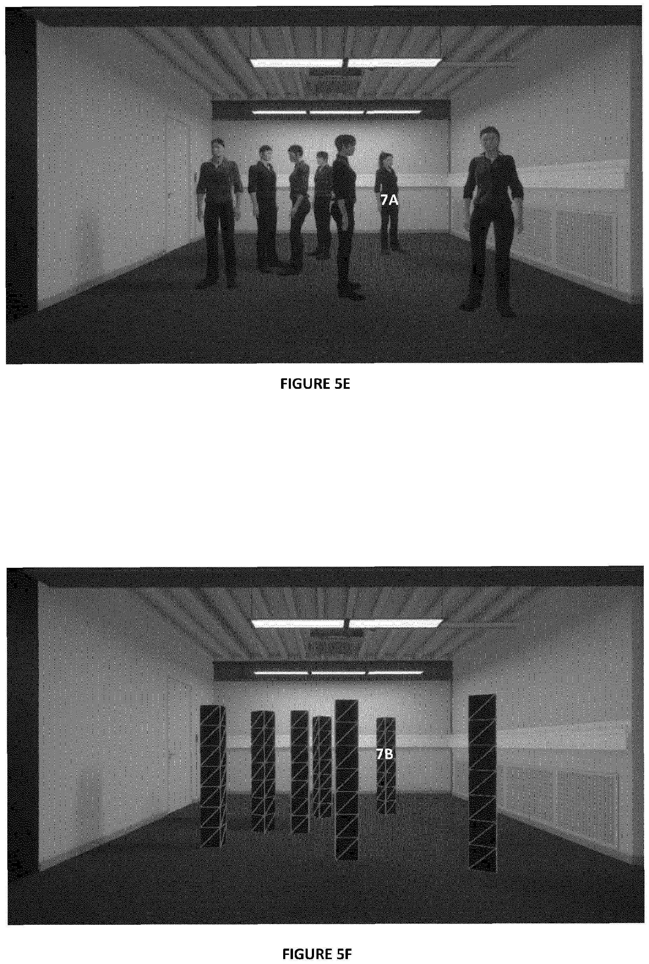

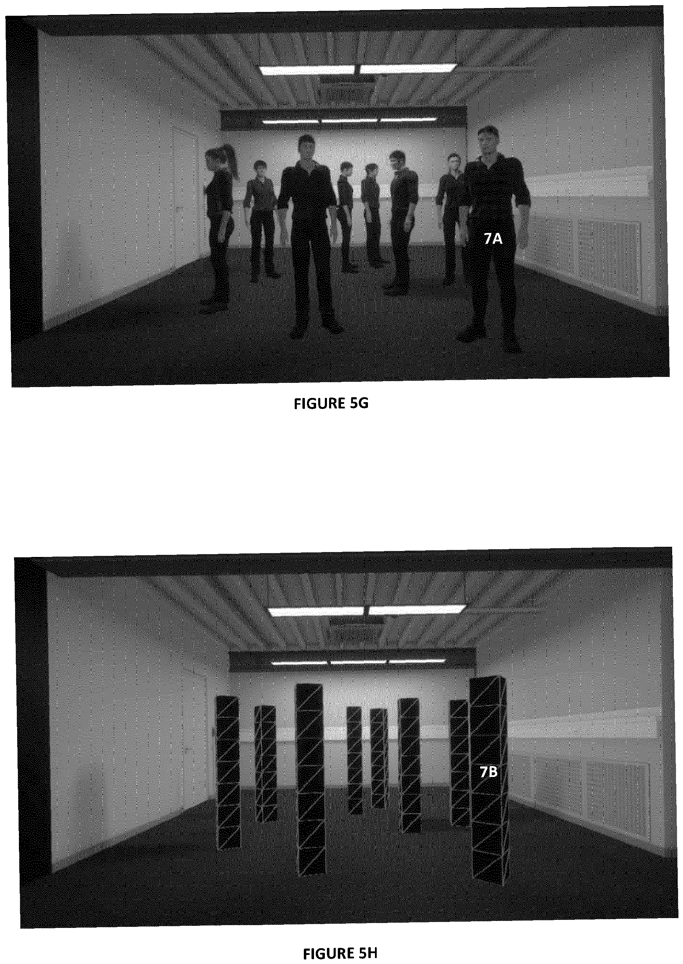

Figure 5A shows an example of the virtual room populated with five 3D models of humans with different appearance but globally similar shapes. In Figure 5B, 3D models of humans are subsequently replaced with 3D models of nonhuman-like digital representations (boxes or elongated columns) located in corresponding positions and orientations. Figure 5C shows an example of the virtual room populated with six 3D models of humans with different appearance but globally similar shapes. In Figure 5D, 3D models of humans are subsequently replaced with 3D models of nonhuman-like digital representations (boxes or elongated columns) located in corresponding positions and orientations. Figure 5E shows an example of the virtual room populated with seven 3D models of humans with different appearance but globally similar shapes. In Figure 5F, 3D models of humans are subsequently replaced with 3D models of nonhuman-like digital representations (boxes or elongated columns) located in corresponding positions and orientations. Figure 5G shows an example of the virtual room populated with eight 3D models of humans with different appearance but globally similar shapes. In Figure 5H, 3D models of humans are subsequently replaced with 3D models of nonhuman-like digital representations (boxes or elongated columns) located in corresponding positions and orientations.

Herein, identical reference numerals are used, where possible, to designate identical elements that are common to the Figures. Also, the images are simplified for illustration purposes and may not be depicted to scale.

### DETAILED DESCRIPTION OF SEVERAL EMBODIMENTS

Figures 1A and 1B show an exemplary numerosity estimation impairment (NEI) measurement systems 1 according to the present disclosure.

The system 1 is, for example, for a diagnosis or prognosis of a neurological disorder, for example, Parkinson's disease (PD).

The system 1 is configured to provide results or data that permits, in association, with the results of other evaluations of the patient to provide a diagnosis or prognosis of a neurological disorder.

The system 1 includes at least one electronic display device 3 configured to present 3D scene representations 5 or 3D scene images 5 comprising a plurality of 3D virtual visual stimulus 7 (see, for example, Figure 3E) to be evaluated by a user of the system 1 and a user of the electronic display device 3.

The system 1 also includes at least one 3D scene generating device 11 operably connected to the electronic display device 3. The 3D scene generating device 11 is configured to provide the 3D scene representations or 3D scene images 5 comprising the plurality of 3D virtual visual stimulus 7 to the electronic display device 3

The system 1 also includes at least one electronic input device 15 configured to receive a user input representing, for each 3D scene representation or 3D scene image 5, a user indicated numerosity (UIN) or a user's estimation of numerosity (UIN) of the presented plurality of virtual visual stimulus 7 of the 3D scene representation 5 or 3D scene image 5.

The system 1 also includes electronic storage means 17 configured to store (i) a predetermined numerosity (PN) of the plurality of 3D virtual visual stimulus 7 for each 3D scene representation or 3D scene image 5, and (ii) the user input representing the user indicated numerosity (UIN) of the presented plurality of 3D virtual visual stimulus 7. The storage means 17 may, for example, comprise semiconductor memory, HDD, or flash memory. The 3D scene generating device 11 may, for example, include the electronic storage means 17 (as shown in Figure 1A & Figure 1B), alternatively or additionally it may be located in another element of the system 1.

The system 1 further includes electronic calculation or computing means 19 configured to determine a numerosity estimation impairment (NEI) by determining an overestimation in numerosity estimations by the user using (i) the predetermined numerosity (PN) of the plurality of 3D virtual visual stimulus 7 for each 3D scene representation or 3D scene image 5 and (ii) the associated user input representing the user indicated numerosity (UIN). The electronic calculation or computing means may, for example, comprise one or more processors or microprocessors. The 3D scene generating device 11 may, for example, include the electronic calculation or computing means 19, alternatively or additionally it may be located in another element of the system 1.

The system 1 may, for example, but not necessarily further include a robotic system or platform 21 (as schematically shown in Figure 1A). The robotic system or platform 21 is, for example, disclosed in US patent applications US2016/0176053 and US2018/0177462 the entire contents of both of which are hereby fully enclosed by reference.

The robotic system or platform 21 is, for example, composed of a commercial haptic interface (available, for example, from Phantom Omni, SensAble Technologies), coupled with a three degree-of-freedom robot in the back. In this non-limiting embodiment, participants sit on a chair and control a front robot situated on a table directly in front of them with their right index finger. The back robot is located to be behind their back and reproduces the movements produced with the front robot with virtually no delay in the synchronous condition, and with a delay (for example, a 500 msec delay) in the asynchronous condition, has been shown to induce presence hallucination (PH). This creates different degrees of sensorimotor conflict between the right-hand movement and the somatosensory feedback on the back.

The system 1 may, for example, include a controller 23 (as schematically shown in Figure 1A). The controller 23 is, for example, configured to control and command operations of the 3D scene generating device 11, as detailed further below.

The electronic input device 15 is configured to receive an estimation of the number of the 3D virtual visual stimuli 7 provided or inputted by the user.

The electronic input device 15 can, for example, comprise a mouse and/or keyboard, or a tactile input permitting to input numerosity estimations (NE). The electronic input device 15 may alternatively, comprise a voice or audio recognition device and a head orientation tracking device configured to position a displayed cursor to a position of a visually displayed value for input via voice or audio recognition.

The electronic input device 15 can alternatively or additionally be configured to measure a physiological reaction of the user. For example, the electronic input device 15 is configured to measure a pupil reaction of the user or an electrocardiogram of the user. The measured pupil reaction signal or the measured electrocardiogram is, for example, processed for example by the processing means 19, to determine the presence of a signal profile(s) or signature(s) associated with a number of virtual visual stimuli 7 estimated by the user.

In one exemplary embodiment of the present disclosure as for example shown in Figure 1A, the electronic display device 3 comprises a virtual reality (VR) system.

The virtual reality (VR) system includes a head mounted display (HMD) system, for example, a commercial head mounted display (HMD) system that is an Oculus Rift CV1 coupled with at least one Oculus Rift Sensor or Oculus Rift S.

The virtual reality (VR) system may also include an auditory system comprising, for example, an auditory headset, such as, a commercial auditory headset as provided by Arctis Wireless Pro.

The 3D scene generating device or graphic system 11 of this embodiment includes, for example, a (laptop) computer (running for example Windows 10) and equipped with a processor 19 (for example, an Intel i7 6700HQ processor), memory 17 (for example, 16Gb Random-Access Memory), and a graphics card (for example, Nvidia GeForce GTX 1060), ensuring a constant display rate (for example, 90Hz display rate) for an experiment or measurement provided to the virtual reality (VR) system of the electronic display device 3.

The virtual reality (VR) system is connected or plugged into the graphic system 11. The experiment or measurement running on the graphic system is, for example, implemented in a game software engine or software framework (for example, Unity 2019.3.13f1), for example stored in the storage means 17 of the 3D scene generating device or graphic system 11.

The virtual environment 5 can be modeled or generated using a 3D computer graphics program, for example, in 3DS max (included in the storage means 17) and provides a realistic representation of an experimental or measurement room in which a user may physically be present when participating in the experiment or evaluation. The virtual scene exposure can, for example, be configured to have a low global brightness. This increases the likelihood of hallucinations, which have often been reported to be triggered in low levels of illumination in patients' daily life.

The controller 23 is, for example, operably connected to the 3D scene generating device or graphic system 11 and is configured to instruct or command experiment control, and to monitor experiment progress. The controller 23 is configured to control the experiment or measurement, and to track progress in real time. It may, for example, be implemented by a mobile-compatible application included in the controller 23 and implemented in a game software engine or software framework (for example, Unity 2019.3.13f1). The controller device 23 may, for example, comprise or be included in a smartphone running, for example, Android (for example, a Samsung Galaxy S8+), and operably connected to the graphic system 11, for example over a wireless connection such as Wi-Fi.

In another exemplary embodiment of the present disclosure implementing online numerosity estimation of humans (NEH) and online numerosity estimation of objects (NEO) evaluation, as for example shown in Figure 1B, the 3D scene generating device 11 comprises or consists of a server 11, and the system 1 concerns a numerosity estimation (NE) task implemented for online numerosity estimation of humans (NEH) and online numerosity estimation of objects (NEO) evaluation and is implemented, for example, using javascript.

On the client side and on the electronic display device 3, implementation is for example through javascript, hypertext markup language (html), and cascading style sheets (css) ; and on server side 11 implementation is for example through hyper text transfer protocol secure (https) server in node.js, nginx as a reverse proxy, running, for example, in two docker containers and is hosted on the server 11, for example, in a dedicated Virtual Machine (1xvCPU, 1GB RAM, 40GB HDD).

The server includes the electronic storage means 17 and the electronic calculation or computing means 19. Data is, for example, registered and stored in the storage means 17 of the server.

The presented pictures presented by the electronic display device 3 can be, for example, 1280 pixels width by 720 pixels height, and measured onscreen, for example, approximately 35.2 x 19.8 cm on a computer version of the electronic display device 3, and 17.6 x 9.9 cm on a tablet version of the electronic display device 3. The electronic display device 3 may, for example, include a computer display, a tablet computer, a smart phone, or a television.

The server 11 and the electronic display device 3 are operatively connected via communications network infrastructure and/or a cloud infrastructure 25. The communications network infrastructure may, for example, include a wired and wireless communications network such as cellular and fibered network. The cloud infrastructure may, for example, be used to perform storage and/or processing on behalf of the server 11 and/or the electronic display device 3.

The system 1 is configured to permit presentation of the 3D scene representations or 3D scene images 5 for a time duration allowing the plurality of 3D virtual visual stimulus 7 to be perceived by the user but not counted by the user. The presentation duration assures a non-countable perception or recognition by the user.

The 3D scene generating device or graphic system 11 is, for example, configured to provide the 3D scene representations or 3D scene images 5 for the necessary time duration, for example, by changing the provided 3D scene representations or 3D scene images 5.

The controller 23 can be, for example, configured to set or change the presentation time duration.

The time duration can be, for example, between 100ms and 500ms, or preferably between 150ms and 250ms. For example, 200ms or 250ms.

The plurality of 3D virtual visual stimulus 7 of each 3D scene representation or 3D scene image 5 may, for example, comprise solely a plurality of human-like digital representations 7A, or may comprise solely a plurality of nonhuman-like digital representations or digital representations of non-human objects 7B.

The human-like digital representations 7A and the nonhuman-like digital representations 7B may, for example, be presented in the 3D scene representation or 3D scene image 5 occupying the same amount space.

The 3D scene representations or 3D scene images 5 may, for example, comprise solely a plurality of human-like digital representations 7A that are subsequently replaced with solely nonhuman-like digital representations 7B located in corresponding positions and orientations.

The plurality of human-like digital representations 7A preferably comprise human-like digital representations of different appearance and/or of different human postures. The different appearance is provided, for example, by different clothing, hair cut/style, gender and coloring/patterning as discussed in more detail below. Some or all of the plurality of human-like digital representations 7A may, for example, be displayed in a displacement action or displacement posture in the 3D scene or representations 5 and/or displayed as exchanging with each other by discussion.

The 3D virtual visual stimuli 7, in each 3D scene representation or 3D scene image 5, can for example be located in front of the viewpoint of the user and are non-fully overlapping or only partially overlapping with respect to one other and are all visible to the user in each 3D scene representation or 3D scene image 5.

The 3D virtual visual stimuli 7 can be, for example, randomly distributed in each 3D scene representation or in each 3D scene image 5. The 3D virtual visual stimuli 7 can be, for example, spatially heterogeneously oriented in each 3D scene representation or in each 3D scene image 5. The 3D virtual visual stimuli 7 can be, for example, located within a 60° horizontal field of view of the user. The 3D virtual visual stimuli 7 can be, for example, located so to be perceived by the user as being between 1.5m and 10m, or between 1.75m and 5.25m in depth from a viewpoint of the user. More details of the 3D virtual visual stimuli 7 presentation are provided below.

The electronic calculation means 19 is configured to determine a numerosity estimation impairment (NEI) by, for example, determining an overestimation in the user estimated numerosity (UIN) of the 3D virtual visual stimuli 7 comprising solely the human-like digital representations 7A and a possible overestimation in the user estimated numerosity (UIN) of the 3D virtual visual stimuli 7 comprising solely nonhuman-like digital representations 7B, and by determining for example a difference between (i) an overestimation in the user estimated numerosity (UIN) of the 3D virtual visual stimuli 7 comprising solely the human-like digital representations 7A and (ii) a possible overestimation (an overestimation may exist, or an overestimation may be absent) in the user estimated numerosity (UIN) of the 3D virtual visual stimuli 5 comprising solely nonhuman-like digital representations 7B.

The electronic calculation means 19 is configured to determine numerosity estimation impairment (NEI) of the user using the recorded user indicated or estimated numerosity (UIN) for a plurality of presented 3D scene representations or 3D scene images 5 each having a predetermined numerosity.

The plurality of presented 3D scene representations or 3D scene images 5 include 3D virtual visual stimuli 7 of different numerosity for solely human-like digital representations 7A and for solely nonhuman-like digital representations 7B, and each numerosity of the plurality of presented 3D scene representations or 3D scene images 5 is reproduced a plurality of times, and may, for example, have a different 3D virtual visual stimuli orientation distribution or positional distribution for each reproduction, or may, for example, have the same 3D virtual visual stimuli orientation distribution or positional distribution for each reproduction, or a mix of both.

Each numerosity of the plurality of presented 3D scene representations or 3D scene images 5 can be, for example, reproduced a plurality of times having a different 3D virtual visual stimuli orientation distribution or positional distribution. The plurality of times of this reproduction can be, for example, between 10 and 100 times for each numerosity.

The electronic calculation means 19 is, for example, configured to obtain from the electronic storage means 17 and/or receive from the electronic input device 15 or the electronic display device 3 the numerosity estimations of the user (UIN) for each predetermined numerosity of both the human-like digital representations 7A and the nonhuman-like digital representations 7B.

The electronic calculation means 19 is, for example, configured to compare the numerosity estimations of the user (UIN) for both the human-like digital representations 7A and the nonhuman-like or object digital representations 7B. The electronic calculation means 19 is configured to determine a numerical score by, for example but not limited to, computing a normalized ratio between the human-like digital representation numerosity estimation scores and the nonhuman-like or object digital representation numerosity estimation scores.

The impairment of numerosity estimation concerns or is represented by a bias or systematic bias for overestimating people 7A, but not objects 7B, when performing numerosity estimation of between at least two different numbers of stimulus elements 7; for example, 7 and 8 stimulus elements as shown in Figure 4 which shows the human asynchronous / human synchronous difference (for experimental results in virtual reality (VR) with robotic induced presence hallucination (PH)).

To compute a score of a subjects' impairment in numerosity estimation, this impairment in numerosity estimation is a numerical score that may, for example, be established by computing the difference between the numerosity estimation of humans (NEH) 7A for each numerosity (for example, 5 to 8) and trial and the numerosity estimation of non-human elements (NEO) 7B for each numerosity (for example, 5 to 8) and trial, and dividing this difference by the sum of the numerosity estimation of humans (NEH) 7A for each numerosity (for example, 5 to 8) and trial and the numerosity estimation of non-human elements (NEO) 7B for each numerosity (for example, 5 to 8) and trial.

The resulting index of bias for overestimating more people than objects can then be used by a medical doctor in relation with other neuropsychological evaluation by linking it to a proneness to Minor Hallucinations and thus to a more rapidly evolving form of the cognitive decline.

The electronic calculation means 19 is configured to determine a numerosity estimation impairment (NEI) by, for example, determining an overestimation in the user estimated numerosity (UIN) of the 3D virtual visual stimuli 7 comprising solely the human-like digital representations 7A and a possible overestimation in the user estimated numerosity (UIN) of the 3D virtual visual stimuli 5 comprising solely nonhuman-like digital representations 7B, and by determining a difference between (i) an overestimation in the user estimated numerosity (UIN) of the 3D virtual visual stimuli 7 comprising solely the human-like digital representations 7A and (ii) a possible overestimation in the user estimated numerosity (UIN) of the 3D virtual visual stimuli 5 comprising solely nonhuman-like digital representations 7B.

The storage means 17 includes, for example, one or more computer programs CP. The computer program or programs CP comprise, for example, program instructions configured, when executed by the electronic calculation means 19, to cause the electronic calculation means 19 to determine the numerosity estimation impairment, as described herein, and/or output or provide the determined numerosity estimation impairment.

The computer program CP may also comprise, for example, program instructions configured, when executed by the electronic calculation means 19, to cause the electronic calculation means 19 to obtain or receive data inputted to the electronic input device 15 or to the electronic display device 3 by a user, for example the inputted numerosity estimations of the user (UIN) for each predetermined numerosity of both the human-like digital representations 7A and the nonhuman-like digital representations 7B, and to record this data in the storage means 17.

The controller 23 is, for example, configured to initiate a user habituation phase (as detailed further below), a black screen display, and a display of a central fixation marker on the electronic display device 3 followed by the presentation of 3D scene representations or 3D scene images 5 comprising the plurality of 3D virtual visual stimulus 7 for which a user indicated numerosity is to be provided. Each 3D scene representation or 3D scene image 5 is presented for a predetermined time duration, as previously mentioned.

The user habituation phase includes, for the virtual reality 3D scene presentation, presenting 3D scene representations 5 comprising solely a plurality of human-like digital representations 7A present in the 3D scene representation 5, or entering/leaving the 3D scene representation 5. Alternatively, solely nonhuman-like digital representations 7B are presented appearing and disappearing in the 3D scene representation 5.

The system 1 and method of the present disclosure thus involve the generation of visual stimuli 7 for the numerosity estimation of humans (NEH) 7A and non-human elements (NEO) 7B in a room 5, and provides a method and algorithm for the random repartition of elements 7 in a room 5 for the generation of stimuli 7 for numerosity estimation (NE), as well as a method and procedure to generate the virtual humans 7A to be used in this stimulus.

The visual stimuli 7 are included, for example, in views in a 3D scene representing a virtual room populated with several elements that can be digital humans or objects (e.g., boxes). Numerosity estimation (NE) is classically done with dots or simple shapes on 2D images (not in a 3D scene) and the approach of the Inventors of the present disclosure is thus very different from the one used by experts in psychophysics.

As mentioned previously, the system 1 and methods for presenting numerosity estimation (NE) stimuli in appropriate conditions include a method to present stimulus in virtual reality (VR) (e.g., on a subject immersed in a virtual environment with a virtual reality (VR) headset) for laboratory testing, as well a method to present stimulus in home-based settings (e.g., on a computer, or television, or tablet, or phone, etc.) for online testing.

In the approach of the Inventors, subjects can for example be shown the visual stimuli 7 either in virtual reality (VR) from an immersive viewpoint inside the virtual room, or with a screen showing an image of the 3D scene viewed from a fixed viewpoint (e.g., computer screen, tablet). This approach of the Inventors is robust to different modes of stimulus presentation, which goes beyond the usual known methods.

The procedures of the present disclosure for presenting stimuli for the reliable estimation of numerosity estimation impairment (NEI) include a procedure to conduct numerosity estimation (NE) testing (trials, repetitions, randomization, etc.), as well as procedures to enable the comparison of numerosity estimation of human (NEH) vs. non-human elements (NEO) (randomization, specific numerosity to test, etc.).

The procedure for numerosity estimation (NE) that is specified in the approach of the Inventors gives very specific indications that are based on experimental validation and that cannot be guessed or obtained easily or obtained by chance thanks to, for example, a specific timing of the presentation of stimulus, specific randomization and repetition of the numerosity estimation (NE) task.

Computing the score of the subjects' impairment in numerosity estimation (human vs. non-human elements) is used as an indicator for diagnostic / prognosis of a neurodegenerative disorder. This approach of the Inventors is different to numerosity estimation (NE) research which usually does not compare different types of elements nor compare the numerosity estimation (NE) for human (NEH) versus the NE for non-human elements (NEO). Moreover, an important originality and innovative aspect of the present disclosure is that numerosity estimation (NE) is usually not linked to other cognitive deficits, hallucinations, or neurodegenerative disorders.

The system 1 of the present disclosure includes, for example in the 3D scene generating device 11, a computer program CP implementing an algorithm or method for the random repartition of elements in a room for the generation of stimuli 7 for numerosity estimation (NE).

The algorithm or computer program CP permits the elements 7, to be evaluated for numerosity estimation (NE), to be placed in the virtual scene 5 in front of the subject's viewpoint in a way that they do not completely overlap when viewed from the viewpoint and the elements 7 are all visible to the user.

Elements 7 are, for example, distributed in the subject's view angles in a range from -90° (left) to 90° (right). Elements 7 may be placed, for example, between 1.5m and 10m or preferably between 1.75m and 5.25m in depth from the viewpoint, and, for example, between -1.5m and 1.5m from right to left.

Elements 7 may occupy, for example, a maximum of 60° horizontal field of view. This ensures that all elements 7 are located within the participants' peripheral field of view, and that they are displayed inside and close to the limit of 30° retinal eccentricity, which is the limit from which the visual acuity decreases more strongly.

The above placing of the elements 7 in the stimulus assures a robust and reliable numerosity estimation (NE) and impairment determination. It should be noted that mere random pictures of people do not satisfy the requirements for the numerosity estimation (NE) task.

An exemplary generation of a virtual scene 5 by the system 1 is shown in Figures 3A to 3E.

In first step (step 1) shown in Figure 3A, initialization and identification of a point of view (Location (point), direction (vector), field of view (FOV, angle)) is performed. This defines the visible area VA of the room as shown in dark gray on the right panel of Figure 3A.

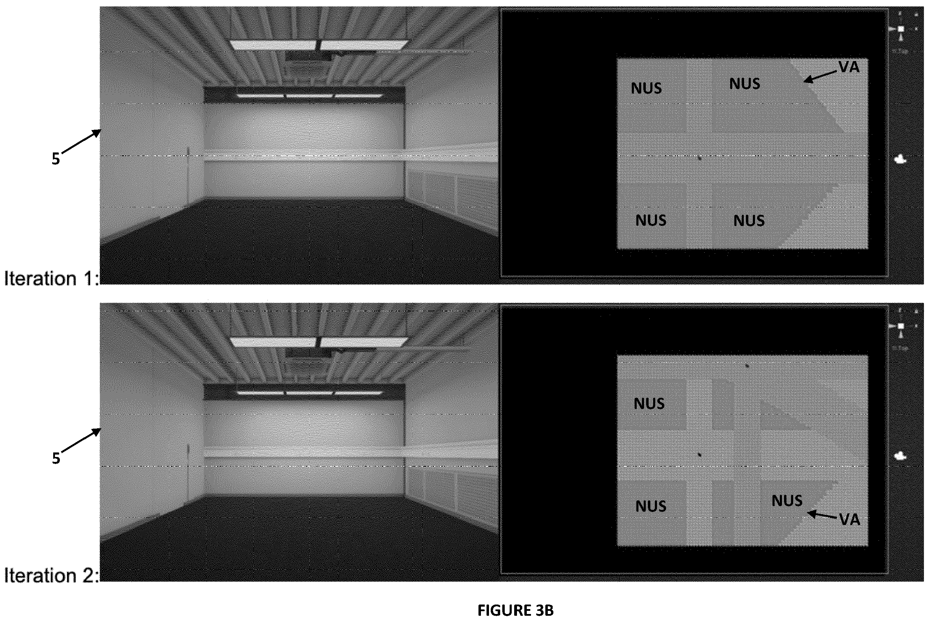

In a further step (step 2), which is repeated for each element 7 to be placed in the room (see Figures 3B to 3D), comprises the finding a location in non-used space NUS and marking space as used by marking an area around a line between viewpoint location and element position, marking an area around a line parallel with viewpoint direction passing by element position, and marking an area around a line perpendicular with viewpoint direction passing by element position.

A further step (step 3) comprises populating the room with chosen stimuli 7, either humanoid figures 7A or objects 7B for all stimuli 7, as shown in Figure 3E.

Further details relating to generation and positioning of such avatars 7A are provided further below.

The system 1 of the present disclosure, for example the 3D scene generating device 11, also includes a computer program CP implementing an algorithm or method to generate the virtual humans 7A and their appearance as presented to the user. The virtual room is populated with 3D models of virtual characters 7A with different appearance but globally similar shapes.

For example, in the case of numerosity estimation NE involving up to 12 elements, 6 different male models and 6 different female models can be used with, for example:
- 3 different haircuts, 4 different pants and 4 different shirts available for male models.
- 5 different haircuts, 3 different pants and 4 different shirts available for female models.

For generating a number N of human elements 7A (for example, for N ≤ 12) for the numerosity estimation NE stimulus, the computer program CP of the system 1 can be, for example, configured to implement one or more or all of the following specifications:
(i) 50%-50% male/female human models (+1 randomly selected male or female when N is odd).
(ii) Each human model 7A is assigned with a randomly chosen haircut, pants and shirt (preferably with no duplicate when possible, then the least amount of duplication possible).
(iii) Colors and properties of each human model 7A is then randomly altered as follows:
   - base haircut color is mixed with a different grey tone color (chosen for example in the interval [0; 0.35f]), from an evenly spaced numbers (number of points equal to the number of desired models), without duplicates.
   - base shirt color is mixed with a different grey tone color (for example, chosen in the interval [0.15f; 0.35f]), from evenly spaced numbers (number of points equal to the number of desired models), without duplicates.
   - base pants color is mixed with a different grey tone color (for example, chosen in the interval [0.05f; 0.3f]), from evenly spaced numbers (number of points equal to the number of desired models), without duplicates.
   - base boots color is mixed with a different grey tone color (for example, chosen in the interval [0.0f; 1.0f]), from evenly spaced numbers (number of points equal to the number of desired models), without duplicates.
   - posture corresponds to a timepoint of a human idle animation (for example, chosen in the interval [0.0f; 1.0f]), from evenly spaced numbers (number of points equal to the number of desired models), no duplicates.

Every element 7, either humanoid figures 7A or objects 7B, shall be entirely visible in the visible area VA of a user, with no overlapping (or minimized partial overlapping when no overlapping is not possible) between the elements 7.

Figure 5A to 5H shows examples of possible configurations of the virtual room populated with 3D models of humans or non-human elements with different appearance but globally similar shapes. Figure 5A shows an example of the virtual room populated with five 3D models of humans with different appearance but globally similar shapes. In Figure 5B, 3D models of humans are subsequently replaced with 3D models of nonhuman-like digital representations (boxes or elongated columns) located in corresponding positions and orientations. Figure 5C shows an example of the virtual room populated with six 3D models of humans with different appearance but globally similar shapes. In Figure 5D, 3D models of humans are subsequently replaced with 3D models of nonhuman-like digital representations (boxes or elongated columns) located in corresponding positions and orientations. Figure 5E shows an example of the virtual room populated with seven 3D models of humans with different appearance but globally similar shapes. In Figure 5F, 3D models of humans are subsequently replaced with 3D models of nonhuman-like digital representations (boxes or elongated columns) located in corresponding positions and orientations. Figure 5G shows an example of the virtual room populated with eight 3D models of humans with different appearance but globally similar shapes. In Figure 5H, 3D models of humans are subsequently replaced with 3D models of nonhuman-like digital representations (boxes or elongated columns) located in corresponding positions and orientations.

The system 1 of Figure 1A is configured to implement a method for presenting numerosity estimation (NE) stimuli 7 in virtual reality VR. This method can, for example, be performed as a laboratory test.

Participant are immersed in virtual reality (VR) with head mounted displays (HMD), and perceive being located inside a virtual experiment room from a given location.

Preferably, participants first experience a habituation phase. The habituation phase may, for example, last 60 secs.

In the case of trials or measurements corresponding to numerosity estimation of humans (NEH) 7A, the habituation phase preferably shows that virtual people can be present in the virtual room with the participant or user, for example, shown entering from a door of the virtual room and/or leaving the virtual room. The human models 7A may be shown to move in the virtual environment, some of them (between 2 and 3 depending on the habituation phase) may also be shown discussing together.

In the case of trials or measurements corresponding to non-human elements 7B, the habituation phase shows for example non-human elements such as vertical boxes appearing (for example, unfolding) and disappearing (for example, folding) in the virtual room. The vertical boxes replacing and matching position and orientation of digital humans may be shown in the virtual room. Several habituation phases may be shown, for example, a total of four different habituation phases can presented to participants before each testing block.

Following the habituation phase, the virtual reality (VR) display turns to black (reproducing a situation as if light in the virtual room was turned off) before doing the numerosity estimation (NE) trial or measurement with the participant or user.

For each numerosity estimation (NE) trial or measurement, an acoustic cue (e.g. double beep, 1000Hz, 250msec each beep separated by 250msec) can be used to indicate the start of a trial or measurement. Participants open their eyes (if closed) and maintain their gaze on a central fixation cross briefly presented (for example, between 500msec and 1500msec) before the presentation of the visual stimulus 7.

The light turns on in the virtual room for a short time duration, for example, 200ms, showing the view of the room populated with many digital humans 7A or virtual non-human elements 7B. Participants then report the number of humans ("How many people are in the room?") or non-humans elements ("How many objects are in the room?") that they estimated to be in the virtual environment. The reporting by the participant can, for example, be performed by the participant targeting a value on a horizontal slider visually presented to the participant in the virtual display, participants control a displayed cursor by turning their head left or right, and may validate audibly by speaking and, for example, saying out loud 'OK' when simultaneously pointing the displayed cursor to or on the desired answer. For example, a number between 0 and 20 is displayed, and validation is performed using voice recognition.

The system 1 of Figure 1B is configured to implement a method for presenting numerosity estimation (NE) stimuli 7 in a home or distance-based setting, that is, outside a laboratory or clinic. This method can, for example, be performed as an online test.

Participants can perform the numerosity estimation (NE) task on a computer or on a tablet 3 by connecting to a website provided by a server 11. The participants may use, for example, a mouse and/or keyboard, or a tactile (finger) input on a smartphone or a tablet to navigate through the presented questionnaire, to answer questions and input numerosity estimations.

The numerosity estimation (NE) trials or measurements include, for example, turning the display to black, showing a fixation cross at the center (briefly presented, for example, between 500msec and 1500msec), and then flashing (for example, at 250ms) images representing a 3D room containing several humans 7A or non-human elements 7B. Participants then report, by for example typing in or touching an integer formatted text box, the number of humans 7A ("How many people were in the virtual room?") or non-human elements 7B ("How many objects were in the virtual room?") they estimated to be in the presented image.

Before starting the task or measurement, participants may be asked to perform a display size calibration procedure, for example participant may be asked to measure with a ruler and report the length (for example, in cm) of a line displayed on their screen 3, which can be used to set a scale factor that causes the stimuli 7 to have a known and precise size for all participants, independently from the different display size and resolution of the participants.

Preferably, the participants are instructed to sit in front of their monitor screen 3 at a distance therefrom of, for example, around 1m on a computer and around 50cm on a smartphone or tablet.

Non-limiting and exemplary procedures for presenting stimuli 7 for a reliable estimation of numerosity estimation impairment (NEI) are now described.

The numerosity estimation (NE) task can be done by repeating several numerosity estimation (NE) trials with different numerosity. The number of elements 7 range from 5 to 8 (e.g., 6 people in the room). These numbers are specifically selected to correspond to the early range of numerosity estimation, without the possibility to count and with classically known performances of human participants.

The participant preferably should do (at least) 10 trials for each numerosity (i.e. total of 40 trials for numerosity 5 to 8). There should thus be 10 different stimuli 7 configurations per numerosity. This means that each visual stimulus (both for human 7A or non-human elements 7B) has a specific configuration in term of elements position, orientation, etc. as previously mentioned above in relation to the generation of visual stimuli.

The order of presentation of the numerosity (for example, in the 40 trials) preferably should be randomized.

To enable a comparison of numerosity estimation of human elements (NEH) 7A vs. non-human elements (NEO) 7B, the estimation of numerosity estimation impairment (NEI) is done by repeating several numerosity estimation (NE) trials with different numerosity for both human (people 7A in the room) and non-human elements (boxes 7B).

The total numerosity estimation impairment (NEI) task is thus separated in two parts, one presenting digital humans 7A in the virtual environment 5 and the other one presenting non-human-like digital representations 7B, for example, boxes 7B in the virtual environment. The order is randomized across participants.

The test preferably includes at least or approximately 10 trials for each numerosity and for both human 7A and non-human 7B digital representations.

The 10 different stimuli configurations generated for each numerosity preferably match or correspond for human 7A and non-human elements 7B. For example, for a given numerosity, the for example 10 configurations shown for humans 7A should correspond to the 10 configurations for non-human elements 7B, in terms of position and orientation.

The above numbers or related trial information is exemplary and should not be considered to restrict the above described procedure.

In relation to the generation and positioning of the human-like digital representations or avatars 7A, more details of an exemplary and non-limiting stimuli generation procedure for avatar generation of the present disclosure are now provided.

Concerning avatar generation, an exemplary total of 6 different male avatars and 6 different female avatars were made available.

An exemplary total of 3 different haircuts, 4 different pants and 4 different shirts were made available for male avatars.

An exemplary total of 5 different haircuts, 3 different pants and 4 different shirts were made available for female avatars.

On generation of a number n of new avatars (n ≤ 12):
- if n is even: n/2 different male avatar were generated, along with n/2 different female avatars are generated
- if n is odd: (n - 1)/2 different male avatar were generated, along with (n - 1)/2 different female avatars were generated. A last avatar is generated, either a male or a female.

Each avatar is assigned with a randomly chosen haircut, pants and shirt (no duplicate as long as possible, then the least duplicate as much as possible).

Each avatar base haircut color is mixed with a different greytone color chosen in the interval [0; 0.35f ], from an evenly spaced numbers (number of points equal to the number of desired avatars), no duplicates.

Each avatar base shirt color is mixed with a different greytone color chosen in the interval [0.15f;0.35f], from an evenly spaced numbers (number of points equal to the number of desired avatars), no duplicates.

Each avatar base pants color is mixed with a different greytone color chosen in the interval [0.05f;0.3f], from an evenly spaced numbers (number of points equal to the number of desired avatars), no duplicates.

Each avatar base boots color is mixed with a different greytone color chosen in the interval [0.0f;1.0f], from an evenly spaced numbers (number of points equal to the number of desired avatars), no duplicates.

Each avatar posture corresponds to a timepoint of a human idle animation chosen in the interval [0.0f;1.0f], from an evenly spaced numbers (number of points equal to the number of desired avatars), no duplicates.

A non-limiting exemplary procedure for avatar positioning can be implemented as indicated below.
1st step: Creation of a 2-dimensional grid representing the possible avatar positions. Exemplary Specifications: depth=6.5m; width=4.5m; spacing=0.05m; position center=(-1.5m,0m).
2nd step: Placement of the camera facing the grid. The camera is, for example, facing the grid on its X axis, 3.5m from the center of the grid and centered in the room on the Z axis (the grid is centered on the Z axis with the room and the camera in later step 6).
3rd step: Calculate the minimal distance between avatar positions on different axis from an orthographic perspective. This value is a spacing measure on different 2D orthographic (normal to X axis, normal to Z axis) and perspective (normal axis from camera to avatar position) axis. For each potential new avatar position, rays are casted on X axis, Z axis, and axis from camera to avatar position. Overlapping positions on the grid with the different rays casted are removed from the list of potential positions for the placement of the next avatar. This value depends on the number of avatar to display, the lower the number of avatar to display, the higher the spacing is.
4th step: Remove positions on the grid that are not seen by the camera. Exemplary Specifications: angle=90°; offset=f min space_between_possible_positions.
5th step: Reduce the grid to the desired one. This allows to remove borders of the grid along with centering the desired zone with the camera. One can crop the grid on 2m on the back, 0.25m on the front, 0.5m on the left and 0.75m on the right. The result is a grid with a depth of 4.25m and a width of 3.25m, centered with the room and the camera on the Z axis.
start_loop_placement:
   6th step: Choose a random position for an avatar in the possible list of positions in the grid.
   7th step: Spacing rule on the perspective view from the camera to the avatar position. For example, a ray of width 2 * *f_ratio_character_occlusion* * *f_min_space_between_possible_positions* is casted on the axis from the camera position to the position of the avatar selected in step 6. Overlapping positions on the grid with the ray casted are removed from the list of potential positions for the placement of the next avatar.
   8th step: Spacing rule on the orthographic view on the X axis.
      For example, a ray of width 2 * *f_min_space_between_possible_positions* is casted on the Z axis from the X position of the avatar selected in step 6. Overlapping positions on the grid with the ray casted are removed from the list of potential positions for the placement of the next avatar.
   9th step: Spacing rule on the orthographic view on the Z axis.
      For example, a ray of width 2 * *f_min_space_between_possible_positions* is casted on the X axis from the Z position of the avatar selected in step 6. Overlapping positions on the grid with the ray casted are removed from the list of potential positions for the placement of the next avatar.
   goto start_loop_placement: (until positioning the desired number of avatars -i_nb_characters ∈ [5; 8] in the exemplary paradigm)
   10th step: Positioning of the avatars.
      The generated avatars of the previously mentioned avatar generation are placed on the different positions selected on iterations of step 6.

Each avatar is rotated of a value chosen in the interval [-90;90], from an evenly spaced numbers (number of points equal to the number of desired avatars), no duplicates.

Implementations described herein are not intended to limit the scope of the present disclosure but are just provided to illustrate possible realizations.

While the invention has been disclosed with reference to certain preferred embodiments, numerous modifications, alterations, and changes to the described embodiments, and equivalents thereof, are possible without departing from the sphere and scope of the invention. Accordingly, it is intended that the invention not be limited to the described embodiments and be given the broadest reasonable interpretation in accordance with the language of the appended claims. The features of any one of the above described embodiments may be included in any other embodiment described herein.

## Claims

1. Numerosity estimation impairment measurement system (1) for diagnostic/prognosis of a neurological disorder, the system (1) comprising:
- at least one electronic display device (3) configured to present 3D scene representations (5) or 3D scene images (5) comprising a plurality of 3D virtual visual stimulus (7) to be evaluated by a user;
- a 3D scene generating device (11) operably connected to the at least one electronic display device (3) and configured to provide 3D scene representations (5) or 3D scene images (5) comprising the plurality of 3D virtual visual stimulus (7) to the at least one electronic display device (3);
- at least one electronic input device (15) configured to receive a user input representing, for each 3D scene representation (5) or 3D scene image (5), a user indicated numerosity (UIN) of the presented plurality of virtual visual stimulus (7) of the 3D scene representation (5) or of the 3D scene image (5);
- electronic storage means (17) configured to store (i) a predetermined numerosity (PN) of the plurality of 3D virtual visual stimulus (7) for each 3D scene representation (5) or 3D scene image (5), and (ii) the user input representing the user indicated numerosity (UIN) of the presented plurality of 3D virtual visual stimulus (7); and
- electronic calculation means (19) configured to determine a numerosity estimation impairment (NEI) by determining an overestimation in numerosity estimations by the user using (i) the predetermined numerosity (PN) of the plurality of 3D virtual visual stimulus (7) for each 3D scene representation (5) or 3D scene image (5) and (ii) the associated user input representing the user indicated numerosity (UIN).

2. System (1) according to claim 1, wherein the system (1) is configured to permit presentation of the 3D scene representations (5) or 3D scene images (5) for a time duration allowing the plurality of 3D virtual visual stimulus (7) to be perceived and not counted by the user, or for a time duration between 100ms and 500ms, or for a time duration between 150ms and 250ms .

3. System (1) according to any one of the previous claims, wherein the plurality of 3D virtual visual stimulus (7) of each 3D scene representation (5) or 3D scene image (5) comprises solely a plurality of human-like digital representations (7A) or solely a plurality of nonhuman-like digital representations (7B).

4. System (1) according to any one of the previous claims, wherein the system (1) is configured to generate the plurality of human-like digital representations (7A) in the 3D scene representations (5) or 3D scene images (5), wherein the plurality of human-like digital representations (7A) comprise human-like digital representations (7A) of different appearance and/or of different posture.

5. System (1) according to any one of claims 2 to 4, wherein the electronic calculation means (19) is configured to determine a numerosity estimation impairment (NEI) by determining an overestimation in the user estimated numerosity of the 3D virtual visual stimuli (7) comprising solely the human-like digital representations (7A) and by determining a difference with the overestimation in the user estimated numerosity of the 3D virtual visual stimuli (7B) comprising solely non-human-like digital representations (7B).

6. System (1) according to any one of the previous claims, wherein the electronic calculation means (19) is configured to determine numerosity estimation impairment (NEI) of the user using the recorded user indicated numerosity (UIN) for a plurality of presented 3D scene representations (5) or 3D scene images (5) each having a predetermined numerosity (PN), wherein said plurality of presented 3D scene representations (5) or 3D scene images (5) include 3D virtual visual stimuli (7) of different numerosity for solely human-like digital representations (7A) and for solely nonhuman-like digital representations (7B), and each numerosity of the plurality of presented 3D scene representations (5) or 3D scene images (5) is reproduced a plurality of times having a different 3D virtual visual stimuli (7) orientation distribution or positional distribution.

7. System (1) according to any one of the previous claims, wherein each numerosity of the plurality of presented 3D scene representations (5) or 3D scene images (5) is reproduced a plurality of times having a different 3D virtual visual stimuli (7) orientation distribution or positional distribution, wherein said plurality of times is between 10 and 100 times for each numerosity.

8. System (1) according to the previous claim 6 to 7, wherein the electronic calculation means (19) is configured to obtain or receive the numerosity estimations (UIN) of the user for each predetermined numerosity of both the human-like digital representations (7A) and the nonhuman-like digital representations (7B), configured to compare the numerosity estimations (UIN) of the user for both the human-like digital representations (7A) and the nonhuman-like digital representations (7B), and configured to determine a numerical score by normalizing the human-like digital representation numerosity estimation scores with the nonhuman-like or object digital representation numerosity estimation scores.

9. System (1) according to anyone of the previous claims, wherein the at least one electronic input device (15) is configured to receive an estimation of the number of the 3D virtual visual stimuli (7) provided by the user.

10. System (1) according to anyone of the previous claims 1 to 9, wherein the at least one electronic input device (15) is configured to measure a physiological reaction of the user; or wherein the at least one electronic input device (15) is configured to measure a pupil reaction of the user or an electrocardiogram of the user.

11. System (1) according to anyone of the previous claims, wherein the 3D scene generating device (11) includes a virtual reality scene generator and the at least one electronic display device (3) includes a head-mounted display; or wherein the 3D scene generating device (11) includes a server and the at least one electronic display device (3) includes a computer display, tablet computer, smart phone, or television.

12. System (1) according to anyone of the previous claims, wherein the 3D virtual visual stimuli (7), in each 3D scene representation (5) or 3D scene image (5), are located in front of a viewpoint of the user and are non-fully overlapping and/or only partially overlapping with respect to one other and are all visible in each 3D scene representation (5) or 3D scene image (5).

13. System (1) according to anyone of the previous claims, wherein the 3D virtual visual stimuli (7) are randomly distributed in each 3D scene representation (5) or in each 3D scene image (5); and/or
wherein the 3D virtual visual stimuli (7) are spatially heterogeneously oriented in each 3D scene representation (5) or in each 3D scene image (5);
and/or
wherein the 3D virtual visual stimuli (7) are located within a 60° horizontal field of view of the user; and/or
wherein the 3D virtual visual stimuli (7) are located so to be perceived by the user as being between 1.5m and 10m, or between 1.75m and 5.25m in depth from a viewpoint of the user.

14. System (1) according to anyone of the previous claims, including at least one controller (23) configured to initiate a user habituation phase, a black screen display, and a display of a central fixation marker on the at least one electronic display device followed by the presentation of 3D scene representations (5) or 3D scene images (5) comprising the plurality of 3D virtual visual stimulus (7) for which a user indicated numerosity (UIN) is to be provided, each 3D scene representation (5) or 3D scene image (5) being presented for a predetermined time duration.

15. System (1) according to the previous claim, wherein the user habituation phase includes, for a virtual reality 3D scene presentation, presenting 3D scene representations (5) comprising solely a plurality of human-like digital representations (7A) present in the 3D scene representation (5) or entering/leaving the 3D scene representation (5); or comprising solely nonhuman-like digital representations (7B) appearing and disappearing in the 3D scene representation (5).
